# EUROPEAN PATENT APPLICATION

(11) **EP 3 903 805 A1**
(43) Date of publication of application: **03.11.2021**
(21) Application number: 19902677.4
(22) Date of filing: 27.12.2019
(51) Int. Cl.: A61K 38/17, A61K 35/17, A61P 35/00, A23L 33/17, G01N 33/68, G01N 33/50

(54) **IMMUNOPOTENTIATING OR ANTICANCER ACTIVITY-AUGMENTING COMPOSITION COMPRISING MAL-EXPRESSED STEM CELL LIKE MEMORY T CELL AS ACTIVE INGREDIENT**

(30) Priority: 28.12.2018 KR 20180172298
(71) Applicant: Neogentc Corp. (Convergence Innovation Building, Asan Institute for Life Sciences, Pungnap-dong), Songpa-gu, Seoul 05505 (KR)
(72) Inventor: HEO, Sun Hee, Seoul 05668 (KR); GONG, Gyungyub, Seongnam-si Gyeonggi-do 13449 (KR); LEE, Hee Jin, Seoul 06003 (KR)
(74) Representative: Schön, Christoph
(86) International application number: PCT/KR2019/018617
(87) International publication number: WO 2020/139027

(57) **Abstract**

The present invention relates to an immunopotentiating or anticancer activity-augmenting composition comprising MAL-expressed, stem cell like memory T cells as an active ingredient. According to the present invention, myelin and lymphocyte (MAL) augments the differentiation, viability, and activity of stem cell like memory cells (Tscm) and MAL-expressed Tscm can be utilized in an immunopotentiating or anticancer activity-augmenting composition. MAL can be advantageously used in a composition for cancer immunotherapy, a composition for induction of Tscm differentiation, a method for screening a cancer immunotherapy agent, a method for screening a Tscm differentiation inducing agent, etc.

## Description

### [Technical Field]

The present invention relates to a composition for immunopotentiating or anticancer activity-augmenting, and more particularly, to a composition for immunopotentiating or anticancer activity-augmenting using stem cell-like memory T (Tscm) cells in which myelin and lymphocyte (MAL) is expressed, a composition for cancer immunotherapy, a composition for inducing Tscm cell differentiation using MAL, and a method for screening a cancer immunotherapeutic agent or a Tscm cell differentiation inducing agent using MAL.

### [Background Art]

According to data from the National Statistical Office, among the causes of death of Koreans, cancer ranks first. In general, cancer is a disease in which cells continue to divide without normal differentiation due to abnormalities in the cycle of cells constituting human tissue, and is generated through three steps of initiation, promotion and progression. The cause of cancer is known as the formation of cancerous tissue by abnormal proliferation due to continuous stimulation of cells in which a normal gene or cancer suppressor gene is mutated by a carcinogen in an environment or food.

Most of the treatments for cancer include surgery, radiation therapy and immunotherapy along with chemotherapy, and methods of studying an anticancer agent for treating cancer include a method of searching for a direct cytotoxic material for cancer cells, a method of searching for a material that regulates the immune ability of an organism, a method of searching for a material that inhibits the metastasis of cancer cells, and a method of searching for a material that inhibits angiogenesis. However, since most anticancer drugs currently used in clinical practice are chemically synthesized materials, problems, such as the causing toxicity to normal cells, are caused, to solve these problems, treatment using an immune response has recently attracted attention.

Immunotherapy, known until 1990, is a non-specific treatment method that boosts immunity in the human body to fight cancer, and includes activated lymphocyte therapy and cytokine therapy using interferon and interleukin. Recently, cancer immunotherapy includes a very broad range of cancer vaccine therapy, monoclonal antibody therapy, adoptive cell therapy and cell therapy. Among the cancer immunotherapy, adoptive cell therapy is a type of passive immunotherapy in which an antitumor effect is expected by administering cells predicted to have immune functions against tumors, and uses various immune cells. Particularly, it is expected that cancer scattered in several places can be effectively removed at one time by the antigen specificity and tissue penetrability of T cells, and since T cells can kill cells specifically expressing an antigen by directly penetrating into tissue through extravasation, they penetrate into various metastatic tissues to remove cancer cells, and therefore the development of cell therapy using T cells is actively progressing.

However, there is a lack of technological development to maintain the survival and activity of administered immune cells *in vivo,* and exhibit sufficient functions as a therapeutic agent. Therefore, in the present invention, among CD3+ T cells, it is intended to use Tscm cells as a way to maintain and increase the survival and activity of immune cells administered *in vivo.*

### [Disclosure]

### [Technical Problem]

The present invention is directed to providing a composition for immunopotentiating or anticancer activity-augmenting.

The present invention is also directed to providing a composition for cancer immunotherapy, or a composition for stem cell-like memory T (Tscm) cell differentiation.

The present invention is directed to providing a method for screening a cancer immunotherapeutic or a Tscm cell differentiation inducing agent.

### [Technical Solution]

To solve the above-described problems, the present invention provides a composition for immunopotentiating or anticancer activity-augmenting comprising a stem cell like memory T cell(Tscm) in which MAL(myelin and lymphocyte) is expressed, as an active ingredient.

The present invention also provides a pharmaceutical composition for cancer immunotherapy or a health functional food composition for cancer immunotherapy comprising an activator for MAL protein expression or activity as an active ingredient.

The present invention also provides a composition for differentiating stem cell like memory T cell comprising an activator for MAL protein expression or activity as an active ingredient.

The present invention also provides a method for screening a cancer immunotherapy agent comprising (a) separating a CD3+ T cell from a sample isolated from a human; (b) differentiating the CD3+ T cell into stem cell like memory T cell by treating with IL-17, IL-15 and test substances and followed by culturing; (c) measuring an expression level of MAL in the stem cell like memory T cell; and (d) selecting a substance having an increased expression or activity level of MAL among the test substances, compared to a control without treating with the test substance.

The present invention also provides a method for screening a stem cell like memory T cell differentiation inducing agent comprising (a) separating a CD3+ T cell from a sample isolated from a human; (b) differentiating the CD3+ T cell into stem cell like memory T cell by treating with IL-17, IL-15 and test substances and followed by culturing; (c) measuring an expression level of MAL in the stem cell like memory T cell; and (d) selecting a substance having an increased expression or activity level of MAL among the test substances, compared to a control without treating with the test substance.

### [Advantageous Effects]

According to the present invention, since myelin and lymphocyte (MAL) improves the *in vivo* differentiation, survival and activation of stem cell-like memory T (Tscm) cells, Tscm cells in which MAL is expressed can be utilized as a composition for immunopotentiating or anticancer activity-augmenting, and the MAL can be effectively used for a composition for cancer immunotherapy, a composition for inducing Tscm cell differentiation, a method for screening a cancer immunotherapy agent, and a method for screening a Tscm cell differentiation inducing agent.

### [Description of Drawings]

FIG. 1 shows the isolation of CD3+ T cells from peripheral blood mononuclear cells.
FIG. 2 shows differentiation into various types of memory T cells using CD3+ T cells.
FIG. 3 shows a gene specifically expressed according to the type of memory T cell.
FIG. 4 is a result of confirming the expression level of MAL according to the type of memory T cell.
FIG. 5 is a result of confirming the numerical decrease in Tscm cells according to the inhibition of MAL expression.
FIG. 6 is a result of confirming the change in *in vivo* lymphocyte survival according to the regulation of MAL expression.

### [Modes of the Invention]

The inventors confirmed that MAL is specifically overexpressed in stem cell-like memory T (Tscm) cells among the various types of memory T cells in differentiation using CD3+ T cells isolated from the blood of a normal person, and the MAL improves the differentiation, survival and activation of Tscm cells, and thus the present invention was completed.

Accordingly, the present invention provides a composition for immunopotentiating or anticancer activity-augmenting comprising a stem cell like memory T cell(Tscm) in which MAL(myelin and lymphocyte) is expressed, as an active ingredient.

The amino acid sequence of the MAL may be represented by SEQ ID NO:1, the base sequence of the MAL may be represented by SEQ ID NO:2.

The stem cell like memory T cell may be differentiated by IL-17 and IL-15, and may be a CD3+CD8+CCR7+CD45RO-CD95+T cell.

Also, the present invention provides a composition for cancer immunotherapy comprising an activator for MAL protein expression or activity as an active ingredient.

The activator for MAL protein expression and activation may be a compound, peptide, peptide mimetic, aptamer, antibody or natural substance, which specifically binds to the MAL protein, but the present invention is not limited thereto.

When the composition of the present invention is a pharmaceutical composition, for administration, other than the above-described active ingredient, a pharmaceutically acceptable carrier, excipient or diluent may be included. The carrier, excipient and diluent may be lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil.

The pharmaceutical composition of the present invention may be formulated into an oral preparation such as a powder, a granule, a tablet, a capsule, a suspension, an emulsion, a syrup or an aerosol, a preparation for external use, a suppository or a sterilized injectable solution. Specifically, for formulation, a surfactant or an excipient such as a filler, a thickener, a binder, a wetting agent, a disintegrant, a diluent, which is generally used, may be prepared. As a solid preparation for oral administration, a tablet, a pill, a powder, a granule or a capsule is used, but the present invention is not limited thereto. Such a solid preparation may be prepared by mixing at least one of excipients, such as starch, calcium carbonate, sucrose, lactose, gelatin and the like, in addition to the active ingredient. In addition, other than simple excipients, lubricants such as magnesium stearate and talc may be used. Other than liquids for oral administration or liquid paraffin, various excipients, such as a wetting agent, a sweetening agent, a flavor, and a preservative may be added in formulation. A formulation for parenteral administration may be a sterilized aqueous solution, a non-aqueous solvent, a suspension, an emulsion, a lyophilizing agent or a suppository. As the non-aqueous solvent or suspension, propylene glycol, polyethylene glycol, a vegetable oil such as olive oil, or an injectable ester such as ethyl oleate may be used. As a suppository base, Witepsol, Macrogol, Tween 61, cacao butter, laurinum, or glycerogelatin may be used.

A suitable dosage of the pharmaceutical composition of the present invention depends on a patient's condition and body weight, severity of a disease, a dosage form, an administration route and duration, but may be selected by those of ordinary skill in the art, and a daily dose of the composition is preferably 0.001 to 50 mg/kg, and if necessary, may be administered once or in divided portions.

In addition, the present invention provides a healthy functional food composition for cancer immunotherapy comprising an activator for MAL protein expression or activity as an active ingredient.

The activator for MAL protein expression and activity may be a compound, peptide, peptide mimetic, aptamer, antibody or natural substance, which specifically binds to the MAL protein, but the present invention is not limited thereto.

When the composition of the present invention is a health functional food composition, various nutrients, vitamins, minerals (electrolytes), flavoring agents including synthetic and natural flavoring agents, coloring agents, fillers (cheese, chocolate, etc.), pectic acid and a salt thereof, alginic acid and a salt thereof, organic acids, protective colloidal thickening agents, pH adjusters, stabilizers, preservatives, glycerin, alcohols, or carbonizing agents used in carbonated beverages may be included. In addition, it may contain pulp for manufacturing natural fruit juices, synthetic fruit juices and vegetable beverages. Such ingredients may be used independently or in combination. In addition, the health food composition may be an any one type of meat, sausage, bread, chocolate, candy, snacks, confectioneries, pizza, ramen, gum, ice cream, various soups, beverages, tea, functional water, drinks, alcoholic beverages and vitamin complexes.

In addition, the health food composition may further include a food additive, and unless specified otherwise, the suitability as a food additive is determined by standards and criteria for a corresponding item in accordance with the general provisions and general test methods in the Korean Food Additives Codex approved by the Ministry of Food and Drug Safety.

The items listed in the Korean Food Additives Codex may be, for example, chemical synthetics such as ketones, glycine, potassium citrate, nicotinic acid and cinnamic acid, natural additives such as a persimmon pigment, a licorice extract, crystalline cellulose, a Kaoliang color and guar gum, and mixed preparations such as L-sodium glutamate, alkali agents for noodles, a preservative formulation and a tar color formulation.

Here, a content of the composition according to the present invention added to food in the process of preparing a health food composition may be suitably added or removed as needed.

In addition, the present invention provides a composition for Tscm cell differentiation, which includes an activator for MAL protein expression or activity as an active ingredient.

The activator for MAL protein expression and activity may be a compound, peptide, peptide mimetic, aptamer, antibody or natural substance, which specifically binds to the MAL protein, but the present invention is not limited thereto.

In addition, the present invention provides a method for screening a cancer immunotherapy agent comprising (a) separating a CD3+ T cell from a sample isolated from a human; (b) differentiating the CD3+ T cell into stem cell like memory T cell by treating with IL-17, IL-15 and test substances and followed by culturing; (c) measuring an expression level of MAL in the stem cell like memory T cell; and (d) selecting a substance having an increased expression or activity level of MAL among the test substances, compared to a control without treating with the test substance.

In addition, the present invention provides a method for screening a stem cell like memory T cell differentiation inducing agent comprising (a) Separating a CD3+ T cell from a sample isolated from a human; (b) differentiating the CD3+ T cell into stem cell like memory T cell by treating with IL-17, IL-15 and test substances and followed by culturing; (c) measuring an expression level of MAL in the stem cell like memory T cell; and (d) selecting a substance having an increased expression or activity level of MAL among the test substances, compared to a control without treating with the test substance.

The expression or activity level of MAL may be measured by any one method selected from a group consisting of reverse transcription-polymerase chain reaction (RT-PCR), real-time PCR, microarray, ELISA, immunoprecipitation, immunohistochemistry, western blot and FACS, but the present invention is not limited thereto.

### Examples

Hereinafter, the present invention will be described in further detail with reference to examples. These examples are merely provided to illustrate the present invention, and it is obvious to those of ordinary skill in the art that scope of the present invention is not limited to these examples according to the gist of the present invention.

### Example 1: Isolation of CD3+ T cells from blood and induction of memory T cell differentiation

### 1-1. PBMC isolation from blood

Blood of four normal people was collected in blood collection tubes, the collected blood was transferred to a 50 ml tube, and the same amount of phosphate buffered saline (PBS, 2% serum) was added to dilute the blood. Afterward, the same amount of a LymphoPrep Solution (Cat NO. 07851, STEMCELL) was slowly injected into the blood from the bottom of the 50 ml tube such that it was not mixed with the diluted blood. Subsequently, following centrifugation at 4 °C and 800 g for 20 minutes, a buffy coat layer was transferred to a fresh tube, PBS (2% serum) was added, and then a supernatant was removed after centrifugation at 400 g. The washing process was repeated three times, peripheral blood mononuclear cells (PBMCs) were recovered to calculate a cell count and then frozen and stored before use in an experiment.

### 1-2. Isolation of CD3+ T cells from PBMCs

CD3-expressing T cells were isolated using PBMCs isolated in Example 1-1. Briefly, CD3-expressing T cells were obtained by methods of removing cells expressing CD14, CD15, CD16, CD19, CD34, CD36, CD56, CD123 and CD235a using a Pan T cell isolation kit (Miltenyi Biotec, 130-096-535).

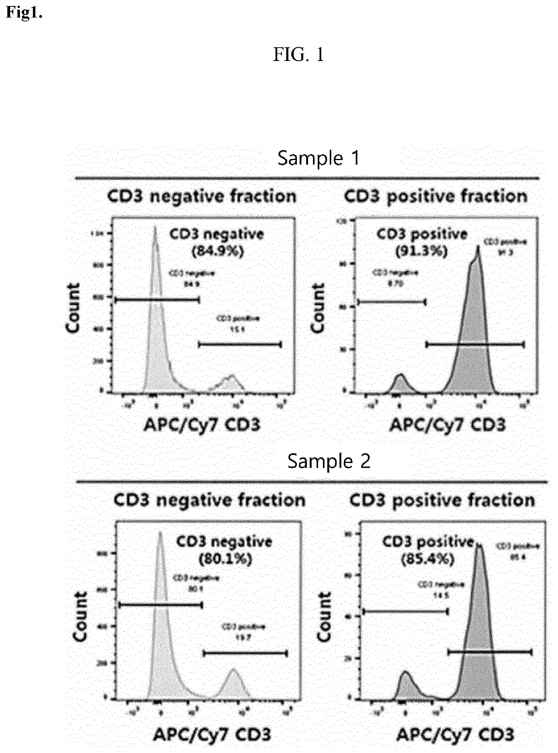

After isolation, as shown in FIG. 1, CD3-expressing T cells were confirmed through FACS analysis, and as a result of measuring isolation efficiency, the CD3-expessing T cells were detected to be 91.3% and 85.4%.

### 1-3. Induction of memory T cell differentiation from CD3+ T cells

The isolated CD3-expressing T cells were seeded in an anti-CD3 antibody-coated plate, and was incubated for 72 hours using a culture solution containing 1 µg /ml of anti-CD28 antibodies. 10 µg/ml IL-7 and 10 µg/ml IL-15 were contained in a medium for inducing differentiation, and a control did not include these interleukins. After 72-hour culture, an increase in Tscm cells was confirmed by FACS analysis.

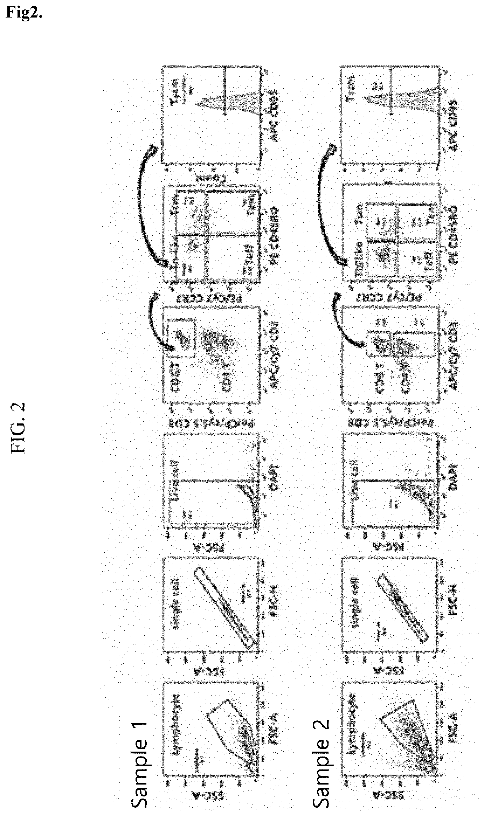

As shown in FIG. 2, through the FACS analysis, CD8-expressing T cells were sorted into naive-like T cells (Tn-like T), central memory T (Tcm) cells, effector T cells (Teff) and effector memory T (Tem) cells, and the Tn-like T was sorted into stem cell-like memory T (Tscm) cells and naive T cells (Tn) and analyzed. It was confirmed that most of the Tn-like T cells are Tscm cells, indicating that differentiation from CD3+ T cells into memory T cells was well induced.

### Example 2: Cell sorting by type of memory T cell and RNA extraction

### 2-1. Cell sorting by type of memory T cell

Finally, the CD8+ T cells, Tscm (CD3+CD8+CCR7+CD45RO-CD95+) cells, Tcm cells and Tern cells were sorted as described in Example 1-3 using an FACS sorter, and then used in an experiment.

### 2-2. RNA extraction

To extract total RNA per type of memory T cell, TRIzol (Invitrogen, Carlsbad, USA) was used. Each cell was put into a 1.5 ml tube, 1.0 ml of TRIzol was added and mixed, 0.2 ml of chloroform was added, followed by reaction at room temperature for 2 minutes. Subsequently, after centrifugation at 13,000 rpm for 15 minutes, 0.5 ml of a supernatant was transferred to a fresh tube. Afterward, 0.5 ml of isopropanol was added and mixed, and reacted at room temperature for 10 minutes, followed by centrifugation at 13,000 rpm for 10 minutes. 75% alcohol was added to the pellet obtained by the centrifugation and centrifuged at 7,500 rpm for 5 minutes, and the resulting pellet was dried in air, and 20 to 50 µl of RNase-free water was added according to an amount of the pellet to lyse the pellet. Afterward, RNA was quantified at a wavelength of 260 nm.

### Example 3: Screening of gene specifically expressed according to the type of memory T cell

### 3-1. mRNA sequencing (Transcriptome)

Extracted RNA was fragmented by fragmentation, and cDNA was synthesized through reverse transcription using a TruSeq Stranded mRNALT Sample Prep Kit and then adapters were attached to both ends thereof. PCR amplification was performed for an amount that enables sequencing, thereby obtaining an insert size of 200 to 400 bp through a size selection process. Sequencing was performed using a HiSeq 2500 system.

### 3-2. Screening of gene specifically expressed according to the type of memory T cell

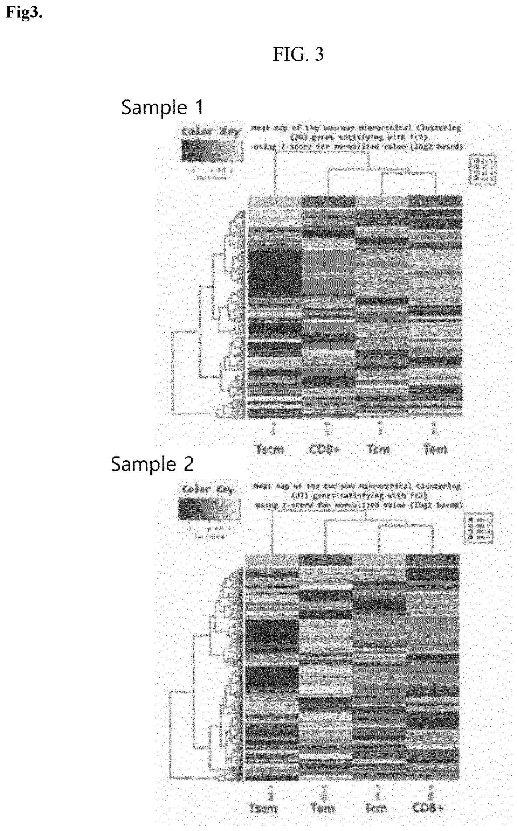

The quality control analysis of raw reads obtained by sequencing was conducted. Basic statistics such as the quality of total reads, total bases, total reads, GC (%) were produced. The reads that had undergone pre-processing were mapped to the reference genome using a HISAT2 program considering a splice, and then aligned reads were generated. Transcript assembly using a StringTie program was carried out, and Fragments Per Kilobase of transcript per Million mapped reads (FRKM) values was calculated using an expression level obtained by the transcript quantification of each sample, which are normalization values considering a read count, a transcript length and a depth of coverage. As a result, as shown in FIG. 3, the expression profile was extracted by comparing the FPKM values of each gene.

### Example 4: Verification of MAL expression specifically expressed in Tscm cells

The expression of the MAL gene increased specifically in Tscm cell was analyzed using the PBMCs collected in Example 1. CD3+ T cells were isolated from PBMCs in Samples 1, 3 and 4 used in transcriptome analysis, and differentiation was induced using IL-7/-15. Afterward, to analyze the expression of the MAL gene according to the type of T cell, quantitative PCR was performed. The expression of the MAL gene was normalized to the expression of GAPDH expressed in each cell type and analyzed.

2 µg of the extracted RNA was synthesized to cDNA using an Omniscript RT kit (Qiagen, 205111). 2 µg of RNA, 0.5 mM dNTP, 1 µM oligo dT, 10 units RNase inhibitor, 4 units RTase, and RT buffer were added to each tube to allow a final volume to be 20 µl, and reacted at 37 °C for 60 minutes, thereby synthesizing cDNA. Real-time PCR was performed with the synthesized cDNA template using a MAL-specific primer, and specific information on the primers used in the experiment is shown in Table 1 below.

**[Table 1]**

| Gene | Forward primer (5' -> 3') | Reverse primer (5' -> 3') |
|---|---|---|
| MAL | GGGTGATGTTCGTGTCTGTG (SEQ ID NO: 3) | ACTGAGGCGCTGAGGTAAAA (SEQ ID NO: 4) |

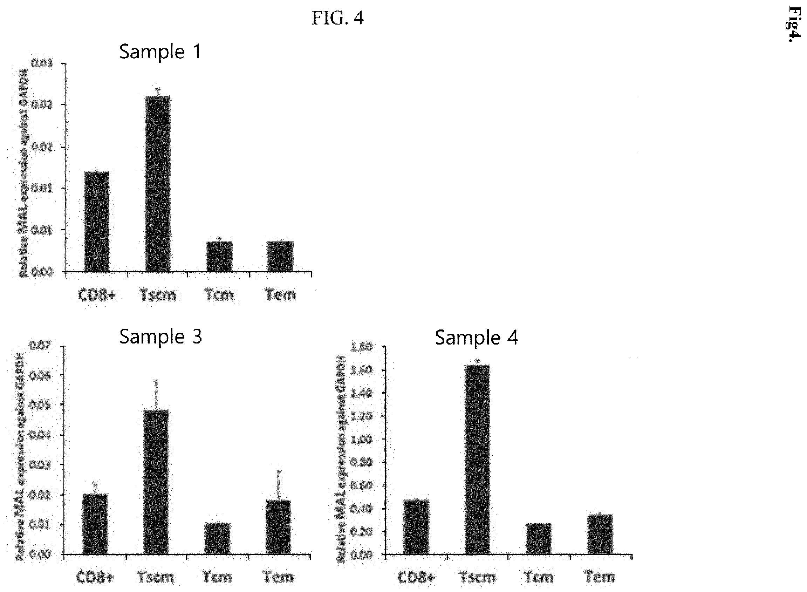

As a result, as shown in FIG. 4, in all of Samples 1 to 3, it was confirmed that the expression of the MAL gene in Tscm cells among the T cell types is considerably increased compared to other T cell types.

### Example 5: Verification of MAL function inducing Tscm cell differentiation

### 5-1. Induction of MAL expression inhibition

According to a method provided by Invitrogen, 50 nM ON-TARGETplus Human MAL siRNA (L-011723-00-0010, Dharmacon, Inc.) or 50 nM ON-TARGETplus Non-targeting Pool (negative control siRNA, siScramble, as a control, not affect any gene in cells, D-001810-10-20, Dharmacon, Inc.) and 1.0 µl Lipofectamine 3000 was mixed in a tube containing an OPTI-MEM solution , and reacted at room temperature for 15 minutes to induce vesicle formation.

Specific information on the MAL siRNA used herein is as follows.
5' - UCAUAAAGCCGCAGUAGAA - 3' (SEQ ID NO: 5)
5' - GCGGUGUUCUCUUUAAUCA - 3' (SEQ ID NO: 6)
5' - CGACUUGCUCUUCAUCUUU - 3' (SEQ ID NO: 7)
5' - CUACAUAGCCACUCUGCUC - 3' (SEQ ID NO: 8)

After the reaction, the vesicle-containing OPTI-MEM was mixed with the culture solution and was treated, and 18 hours later, the medium was replaced with a fresh medium. After 48 hours, RNA was extracted from the cells, and MAL expression was confirmed by the method described in Example 4.

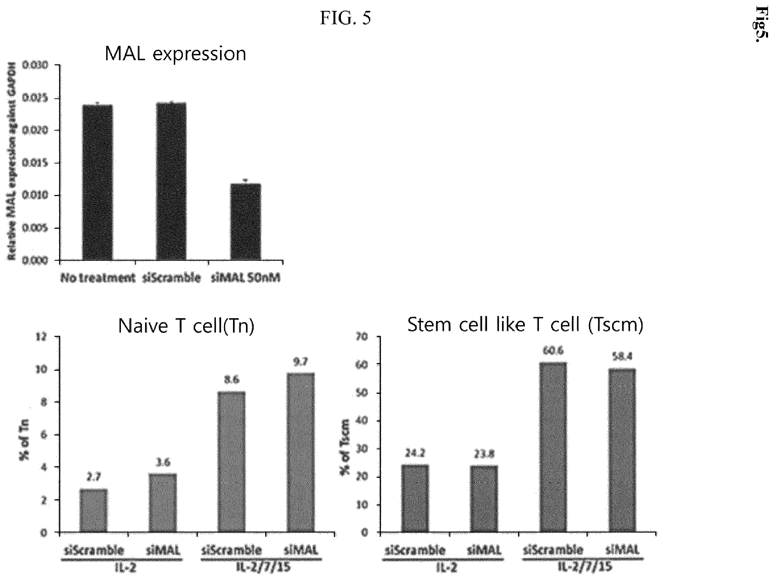

As a result, referring to FIG. 5, compared with an untreated sample and a sample treated with siScramble, in a sample treated with siRNA for MAL, it was confirmed that MAL expression is reduced by more than half.

### 5-2. Memory T cell type analysis according to inhibition of MAL expression

By the method described in Example 5-1, siRNA was treated, and by the method described in Example 1-3, differentiation from CD3+ T cells to memory T cells was induced. After differentiation was induced for 72 hours, the distribution of stem cell-like memory T (Tscm) cells and naive T cells (Tn) was analyzed by FACS analysis.

As a result, as the result shown in FIG. 5 below, it was confirmed that the number of Tscm cells differentiated from Tn cells is reduced according to the decrease in MAL expression.

The result can also be confirmed by an increase in cells that could not relatively differentiate into Tscm cells and that maintain a Tn cell state.

### Experimental Example 6: Verification of MAL function regulating in vivo lymphocyte survival

### 6-1. Confirmation of cloning and expression for inducing MAL overexpression

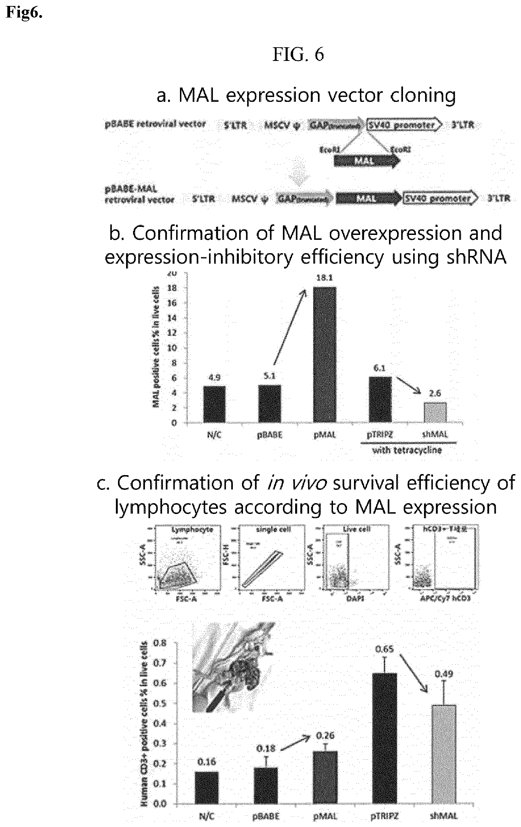

To produce a retrovirus expressing the MAL gene, a pBABE retroviral vector was used as a backbone vector. As shown in FIG. 6A, the MAL gene amplified by PCR was inserted into an EcoRI restriction site of the pBABE vector. A retrovirus was produced in Plat-A cells using the cloned plasmid (pBABE-MAL, pMAL), and the MAL expression inducing efficiency obtained thereby was confirmed by FACS analysis as shown in FIG. 6B. Here, a retrovirus (pBABE) made using the pBABE vector was used as a control. In addition, the group that was not treated with any virus was used as a negative control (N/C).

### 6-2. Induction of inhibition of MAL expression using shRNA

Lentiviruses were produced in HEK293 cells using Human MAL TRIPZ Lentiviral shRNA (RHS4696-200762536, Dharmacon, Inc.) and a non-silencing-TRIPZ Lentiviral shRNA control (RHS4743, Dharmacon, Inc.) vector as a control. The pTRIPZ vector induced shRNA expression by tetracycline treatment, and CD3+ cells were transfected with the produced viruses, and then the medium was replaced with a tetracycline-containing medium. The MAL expression-inhibitory efficiency was confirmed by FACS analysis as shown in FIG. 6B.

### 6-3. Verification of in vivo lymphocyte survival

6-week-old lymphocyte-free NOD-SCID mice were used. The CD3-expressing T cells obtained by the method as described in Examples 1-1 and 1-2 were induced to have MAL overexpression or inhibition using the MAL expressing regulatory systems of Example 6-1 and 6-2. Three days after 1×10⁶ cells per mouse were injected, blood was recovered from the femoral vein. Red blood cells were removed from the recovered blood, and stained using the APC/Cy7 anti-human CD3 Antibody (Cat # 300318, BioLegend, Inc.), and subjected to FACS analysis as shown in FIG. 6C. CD3-expressing T cells of an untreated human were used as a negative control (N/C).

As a result, as the following result shown in FIG. 6C, it was confirmed that the survival of the cells injected *in vivo* increased according to the increase in MAL expression, whereas the decrease in expression reduces *in vivo* survival of the cells.

From the above results, it was confirmed that MAL plays a role in the *in vivo* survival of lymphocytes.

As above, as specific parts of the specification have been described in detail, although it is clear to those skilled in the art that this specific technique is merely a preferred embodiment, the scope of the specification is not limited thereto. Thus, the substantial scope of the specification will be defined by the accompanying claims and their equivalents.

The scope of the present invention is represented by the following claims, and all changes or modifications derived from the meaning and scope of the claims and their equivalent concepts should be interpreted as being included in the scope of the present invention.

## Claims

1. A composition for immunopotentiating or anticancer activity-augmenting comprising a stem cell like memory T cell(Tscm) in which MAL(myelin and lymphocyte) is expressed, as an active ingredient.

2. The composition of claim 1, wherein the amino acid sequence of the MAL is represented by SEQ ID NO:1.

3. The composition of claim 1, wherein the base sequence of the MAL is represented by SEQ ID NO:2.

4. The composition of claim 1, wherein the stem cell like memory T cell is a CD3+, CD8+, CCR7+, CD45RO-, and CD95+T cell.

5. The composition of claim 1, wherein the stem cell like memory T cell is differentiated by IL17 and IL-15.

6. A pharmaceutical composition for cancer immunotherapy comprising an activator for MAL protein expression or activity as an active ingredient.

7. A health functional food composition for cancer immunotherapy comprising an activator for MAL protein expression or activity as an active ingredient.

8. A composition for differentiating stem cell like memory T cell comprising an activator for MAL protein expression or activity as an active ingredient.

9. A method for screening a cancer immunotherapy agent comprises:
(a) separating a CD3+ T cell from a sample isolated from a human;
(b) differentiating the CD3+ T cell into stem cell like memory T cell by treating with IL-17, IL-15 and test substances and followed by culturing;
(c) measuring an expression level of MAL in the stem cell like memory T cell; and
(d) selecting a substance having an increased expression or activity level of MAL among the test substances, compared to a control without treating with the test substance.

10. The method of claim 9, wherein the expression or activity level of MAL is measured by any one method selected from a group consisting of reverse transcription-polymerase chain reaction (RT-PCR), real-time PCR, microarray, ELISA, immunoprecipitation, immunohistochemistry, western blot and FACS.

11. A method for screening a stem cell like memory T cell differentiation inducing agent comprises:
(a) Separating a CD3+ T cell from a sample isolated from a human;
(b) differentiating the CD3+ T cell into stem cell like memory T cell by treating with IL-17, IL-15 and test substances and followed by culturing;
(c) measuring an expression level of MAL in the stem cell like memory T cell; and
(d) selecting a substance having an increased expression or activity level of MAL among the test substances, compared to a control without treating with the test substance.

12. The method of claim 11, wherein the expression or activity level of MAL is measured by any one method selected from a group consisting of reverse transcription-polymerase chain reaction (RT-PCR), real-time PCR, microarray, ELISA, immunoprecipitation, immunohistochemistry, western blot and FACS.


<110>    University of Ulsan Foundation For Industry Cooperation
         THE ASAN FOUNDATION

<120>    Composition for improving immunity reinforcement or anti-cancer
         activity comprising MAL expressed stem cell like memory T cell

<130>    ADP-2019-0582

<160>    8

<170>    KoPatentIn 3.0

<210>    1
<211>    153
<212>    PRT
<213>    Homo sapiens

<400>    1
Met Ala Pro Ala Ala Ala Thr Gly Gly Ser Thr Leu Pro Ser Gly Phe
  1               5                  10                  15

Ser Val Phe Thr Thr Leu Pro Asp Leu Leu Phe Ile Phe Glu Phe Ile
             20                  25                  30

Phe Gly Gly Leu Val Trp Ile Leu Val Ala Ser Ser Leu Val Pro Trp
         35                  40                  45

Pro Leu Val Gln Gly Trp Val Met Phe Val Ser Val Phe Cys Phe Val
     50                  55                  60

Ala Thr Thr Thr Leu Ile Ile Leu Tyr Ile Ile Gly Ala His Gly Gly
 65                  70                  75                  80

Glu Thr Ser Trp Val Thr Leu Asp Ala Ala Tyr His Cys Thr Ala Ala
                 85                  90                  95

Leu Phe Tyr Leu Ser Ala Ser Val Leu Glu Ala Leu Ala Thr Ile Thr
            100                 105                 110

Met Gln Asp Gly Phe Thr Tyr Arg His Tyr His Glu Asn Ile Ala Ala
        115                 120                 125

Val Val Phe Ser Tyr Ile Ala Thr Leu Leu Tyr Val Val His Ala Val
    130                 135                 140

Phe Ser Leu Ile Arg Trp Lys Ser Ser
145                 150


<210>    2
<211>    1153
<212>    DNA
<213>    Homo sapiens

<400>    2
tcttctgccc cgggctcccc tgctcttaac ccgcgcgcgg gggcgcccag gccactgggc        60

tccgcggagc cagcgagagg tctgcgcgga gtctgagcgg cgctcgtccc gtcccaaggc       120

cgacgccagc acgccgtcat ggcccccgca gcggcgacgg ggggcagcac cctgcccagt       180

ggcttctcgg tcttcaccac cttgcccgac ttgctcttca tctttgagtt tatcttcggg       240

ggcctggtgt ggatcctggt ggcctcctcc ctggtgccct ggcccctggt ccagggctgg 300

gtgatgttcg tgtctgtgtt ctgcttcgtg gccaccacca ccttgatcat cctgtacata 360

attggagccc acggtggaga gacttcctgg gtcaccttgg acgcagccta ccactgcacc 420

gctgccctct tttacctcag cgcctcagtc ctggaggccc tggccaccat cacgatgcaa 480

gacggcttca cctacaggca ctaccatgaa aacattgctg ccgtggtgtt ctcctacata 540

gccactctgc tctacgtggt ccatgcggtg ttctctttaa tcagatggaa gtcttcataa 600

agccgcagta gaacttgagc tgaaaaccca gatggtgtta actggccgcc ccactttccg 660

gcataacttt ttagaaaaca gaaatgccct tgatggtgga aaaaagaaaa caaccacccc 720

cccactgccc aaaaaaaaaa gccctgccct gttgctcgtg ggtgctgtgt ttactctccc 780

gtgtgccttc gcgtccgggt tgggagcttg ctgtgtctaa cctccaactg ctgtgctgtc 840

tgctagggtc acctcctgtt tgtgaaaggg gaccttcttg ttcgggggtg ggaagtggcg 900

accgtgacct gagaaggaaa gaaagatcct ctgctgaccc ctggagcagc tctcgagaac 960

tacctgttgg tattgtccac aagctctccc gagcgcccca tcttgtgcca tgttttaagt 1020

cttcatggat gttctgcatg tcatggggac taaaactcac ccaacagatc tttccagagg 1080

tccatggtgg aagacgataa ccctgtgaaa tactttataa aatgtcttaa tgttcaaaaa 1140

aaaaaaaaaa aaa 1153

<210> 3
<211> 20
<212> DNA
<213> Artificial Sequence

<220>
<223> MAL forward primer

<400> 3
gggtgatgtt cgtgtctgtg 20

<210> 4
<211> 20
<212> DNA
<213> Artificial Sequence

<220>
<223> MAL reverse primer

<400> 4
actgaggcgc tgaggtaaaa 20

<210> 5
<211> 19
<212> RNA
<213> Artificial Sequence

<220>
<223>    MAL siRNA


<400>    5
ucauaaagcc gcaguagaa                                                    19


<210>    6
<211>    19
<212>    RNA
<213>    Artificial Sequence

<220>
<223>    MAL siRNA


<400>    6
gcgguguucu cuuuaauca                                                    19


<210>    7
<211>    19
<212>    RNA
<213>    Artificial Sequence

<220>
<223>    MAL siRNA


<400>    7
cgacuugcuc uucaucuuu                                                    19


<210>    8
<211>    19
<212>    RNA
<213>    Artificial Sequence

<220>
<223>    MAL siRNA


<400>    8
cuacauagcc acucugcuc                                                    19